(19) Europäisches Patentamt
European Patent Office
Office européen des brevets

(11) Veröffentlichungsnummer: **0 382 055 A1**

# (12) EUROPÄISCHE PATENTANMELDUNG

(21) Anmeldenummer: **90101791.3**

(22) Anmeldetag: **30.01.90**

(51) Int. Cl.[5]: **C07D 487/08, //(C07D487/08, 241:00,241:00)**

(30) Priorität: **08.02.89 DE 3903622**

(43) Veröffentlichungstag der Anmeldung: **16.08.90 Patentblatt 90/33**

(84) Benannte Vertragsstaaten: **BE DE ES FR GB NL**

(71) Anmelder: **BASF Aktiengesellschaft**
**Carl-Bosch-Strasse 38**
**D-6700 Ludwigshafen(DE)**

(72) Erfinder: **Hesse, Michael, Dr.**
**An der Froschlache 3**
**D-6700 Ludwigshafen(DE)**
Erfinder: **Lermer, Helmut, Dr.**
**Bockenheimer Strasse 12**
**D-6700 Ludwigshafen(DE)**
Erfinder: **Steck, Werner, Dr.**
**Auerstrasse 4**
**D-6700 Ludwigshafen(DE)**
Erfinder: **Fischer, Roman, Dr.**
**Deidesheimer Strasse 1**
**D-6710 Frankenthal(DE)**
Erfinder: **Müller, Herbert, Dr.**
**Carostrasse 53**
**D-6710 Frankenthal(DE)**
Erfinder: **Scharf, Emil, Dr.**
**Mohnstrasse 51**
**D-6700 Ludwigshafen(DE)**

## (54) Verfahren zur Herstellung von 1,4-Diazabicyclo-2,2,2-octan.

(57) Verfahren zur Herstellung von 1,4-Diazabicyclo-2,2,2-octan der Formel I

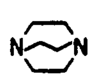

(I),

dadurch gekennzeichnet, daß man 1,2-Diaminoethan und 0 bis 200 Mol% Piperazin an Aluminium-, Bor-, Gallium- und/oder Eisensilikatzeolithen bei Temperaturen von 50 bis 600 °C und Drücken von 0,01 bis 50 bar umsetzt.

EP 0 382 055 A1

## Verfahren zur Herstellung von 1,4-Diazabicyclo-2,2,2-octan

Die vorliegende Erfindung betrifft ein neues und verbessertes Verfahren zur Herstellung von 1,4-Diazabicyclo-2,2,2-octan durch Umsetzung von 1,2-Diaminoethan gegebenenfalls in Gegenwart von Piperazin an Aluminium-, Bor-, Gallium- und/oder Eisensilikatzeolithen bei erhöhten Temperaturen.

Aus der US-A-3 297 701 und der EP-A-111 928 ist die Herstellung von 1,4-Diazabicyclo-2,2,2-octan an Metallphosphaten aus Ethanolamin oder 1,2-Diaminoethan (Ethylendiamin) in minimalen Ausbeuten bekannt.

Aus der DE-A-17 45 627 ist die Herstellung von 1,4-Diazabicyclo-2,2,2-octan aus linearen 1,ω-Aminoethylaminen verschiedener Kettenlänge an sauren Kieselsäure-Tonerde-Katalysatoren in unbefriedigenden Ausbeuten.

Aus der EP-A-150 558 sowie GB-A-2 090 157, GB-A-2 090 158, GB-A-2 090 238, GB-A-2 090 267 und GB-A-2 090 268 ist bekannt, daß beim Einsatz von Substanzen mit nur einer Ethyleneinheit, wie z.B. Ethylendiamin oder Ethanolamin, hauptsächlich lineare Produkte oder einfache Heterocyclen entstehen, wenn als Katalysatoren Metalloxide oder Phosphate eingesetzt werden.

Die bekannten Verfahren liefern unbefriedigende Ausbeuten und/oder Selektivitäten und/oder es treten unerwünschte, schwer oder unvollständig abtrennbare Nebenprodukte auf.

Der Erfindung lag daher die Aufgabe zugrunde, einen besseren Zugang zu den 1,4-Diazabicyclo-2,2,2-octan zu finden und den Nachteilen der bekannten Verfahren abzuhelfen.

Demgemäß wurde ein neues und verbessertes Verfahren zur Herstellung von 1,4-Diazabicyclo-2,2,2-octan gefunden, welches dadurch gekennzeichnet ist, daß man 1,2-Diaminoethan und 0 bis 200 Mol% Piperazin an Aluminium-, Bor-, Gallium- und/oder Eisensilikatzeolithen bei Temperaturen von 50 bis 600 °C und Drücken von 0,01 bis 50 bar umsetzt.

1,4-Diazabicyclo-2,2,2-octan I ist nach folgender Methode erhältlich:

Die Umsetzung erfolgt durch thermische Behandlung von 1,2-Diaminoethan und 0 bis 200 Mol% Piperazin an Aluminium-, Bor-, Gallium- und/oder Eisen silikatzeolithen. Die Reaktion kann sowohl in der Flüssigphase als auch diskontinuierlich oder vorzugsweise kontinuierlich in der Gasphase bei 50 bis 600 °C und 0,01 bis 50 bar durchgeführt werden.

Die Flüssigphasenreaktion kann beispielsweise als Suspensions-, Rieseloder Sumpfreaktion bei Temperaturen von 50 bis 400 °C und Drücken von 0,5 bis 20 bar, vorzugsweise bei Temperaturen von 100 bis 300 °C und Drücken von 1 bis 5 bar durchgeführt werden.

Die bevorzugte Gasphasenreaktion kann beispielsweise bei Temperaturen von 100 bis 600 °C, vorzugsweise bei 150 bis 500 °C und Drücken von 0,1 bis 50 bar, besonders bevorzugt bei 200 bis 400 °C und Drücken von 0,5 bis 5 bar, vor allem bei Atmosphärendruck durchgeführt werden. Bei der Umsetzung in der Gasphase hält man vorteilhaft eine Katalysatorbelastung von 0,1 bis 20, insbesondere von 1 bis 10 g Ausgangsstoff je g Katalysator und Stunde ein (WHSV = g Einsatzgemisch/g Katalysator und Stunde). Die Gasphasenreaktion kann in einem Festbett oder in einem Wirbelbett ausgeführt werden.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bevorzugt werden gasförmigen Reaktionsprodukte sofort in eine Trennung eingebracht und z.B. in einer Fraktionierkolonne in ihre Einzelkomponenten zerlegt.

Die Umsetzung kann aber auch mit Lösungs- oder Verdünnungsmitteln durchgeführt werden. Als solche eignen sich organische Lösungs- oder Verdünnungsmittel wie acyclische oder cyclische Ether mit 2 bis 12 Kohlenstoffatomen, wie Dimethylether, Diethylether, Dibutylether, Dipropylether, Dipentylether oder deren Isomere und THF, Pyran oder Lactone, Polyether, wie Monoglyme, Diglyme, Triglyme, aromatische oder aliphatische Kohlenwasserstoffe wie Benzol, Toluol, Xylol, Pentan, Cyclopentan, Hexan und Petrolether oder deren Gemische und besonders auch Wasser oder wäßrige organische Lösungs- oder Verdünnungsmittel der oben genannten Art.

Die Menge an Piperazin bezogen auf 1,2-Diaminoethan liegt zwischen 0 und 200 Mol% bevorzugt zwischen 30 und 150 Mol%, besonders bevorzugt zwischen 50 und 100 Mol%. Das Molverhältnis von organischen Lösungs- oder Verdünnungsmittel zu den Edukten kann 0 bis 50:1, bevorzugt 0 bis 15:1, besonders bevorzugt 0 betragen.

Als Verdünnungsmittel für die Gasphase eignen sich auch Inertgase wie Stickstoff oder Argon.

Als Katalysatoren für das erfindungsgemäßen Verfahren verwendet man Zeolithe, zweckmäßig in der aciden Form. Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1 : 2. Die Elektrovalenz der

Aluminium enthaltenden Tetraeder ist durch Einschluß von Kationen in den Kristall, z. B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z. B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit-bzw. Chabasit-Typ oder Zeolithe vom Faujasit-Typ, z. B. Y-, X- oder L-Zeolithe.

In diese Gruppe von Zeolithen gehören auch die sogenannten "ultrastabilen" Zeolithe des Faujasittyps, d. h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch eine hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Aluminium-, Bor-, Eisen-, Beryllium-, Gallium-, Chrom-, Arsen-, Antimon- und Wismutsilikatzeolithe oder deren Gemische sowie Aluminium-, Bor-, Gallium- und Eisengermanatzeolithe oder deren Gemische. Insbesondere eignen sich die Aluminium-, Bor- und Eisensilikatzeolithe des Pentasiltyps für das erfindungsgemäße Verfahren. Der Aluminiumsilikatzeolith wird z. B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP-A-34 727 und EP-A-46 504. Die erhaltenen Aluminiumsilikatzeolithe enthalten je nach Wahl der Einsatzstoffmengen ein $SiO_2/Al_2O_3$-Verhältnis von 10 bis 40 000. Aluminiumsilikatzeolithe des Pentasiltyps können auch in etherischem Medium wie Diethylenglykoldimethylether, in alkoholischem Medium wie Methanol bzw. 1,4-Butandiol oder in Wasser synthetisieret werden.

Zu den erfindungsgemäß verwendbaren siliciumreichen Zeolithen ($SiO_2/Al_2O_3 \geq 10$) gehören auch die verschiedenen ZSM-Typen, Ferrierit, Nu-1 und Silicalit®.

Borsilikatzeolithe kann man z. B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP-A-34 727 und EP-A-46 504. Solche Borsilikatzeolithe können ebenfalls hergestellt werden, wenn man die Reaktion statt in wäßriger Aminlösung in etherischer Lösung, z. B. Diethylenglykoldimethylether oder in alkoholischer Lösung, z. B. 1,6-Hexandiol durchführt.

Den Eisensilikatzeolith erhält man z. B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Aluminium-, Bor- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminiumsilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Aluminium- bzw. Borsilikatzeolith direkt nach der Trocknung verformt wird und erst nach der Verformung einer Calcinierung unterworfen wird. Die hergestellten Aluminium- und Borsilikatzeolithe können in reiner Form, ohne Binder, als Stränge oder Tabletten eingesetzt werden, wobei als Verstrangungs- oder Peptisierungshilfsmittel, z. B. Ethylcellulose,

Stearinsäure, Kartoffelstärke, Ameisensäure, Oxalsäure, Essigsäure, Salpetersäure, Ammoniak, Amine, Silikoester und Graphit oder deren Gemische verwendet werden. Liegt der Zeolith aufgrund der Art seiner Herstellung nicht in der katalytisch aktiven, aciden H-Form vor, sondern z. B. in der Na-Form, dann kann diese durch Ionenaustausch, z. B. mit Ammoniumionen und anschließende Calcinierung oder durch Behandlung mit Säuren vollkommen oder partiell in die gewünschte H-Form überführt werden.

Um eine möglichst hohe Selektivität, hohen Umsätzen sowie lange Standzeiten zu erreichen, ist es vorteilhaft, die Zeolithe zu modifizieren. Eine geeignete Modifizierung der Katalysatoren besteht z. B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Ba, Metalle der 3., 4. und 5. Hauptgruppe wie B, Al, Ga, Ge, Sn, Pb, Bi, Seltene Erdmetalle wie La, Ce, Pr, Nd, Er, Yb und U eingesetzt. Die Modifizierung mit diesen Metallen kann durch Ionenaustausch oder Imprägnierung erfolgen.

Zweckmäßigerweise führt man diese Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z. B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Im einzelnen geht man vorteilhaft, z. B. so vor, daß man Zeolithe in Pulverform mit 1 n Phosphorsäure 1 Stunde bei 80 °C behandelt. Nach der Behandlung wird mit Wasser gewaschen, bei 110 °C/16 h getrocknet und bei 500 °C/20 h calciniert. Nach einer anderen Arbeitsweise behandelt man Zeolithe vor oder nach ihrer Verformung mit Bindemittel, z. B. 1 bis 2 h bei Temperaturen von 60 bis 80 °C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400 °C bis 500 °C calciniert.

Wenn bei der erfindungsgemäßen Verwendung der zeolithischen Katalysatoren eventuell eine durch Koksabscheidung bedingte Desaktivierung eintritt, empfiehlt es sich, diese durch Ab-brennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Herstellung der Katalysatoren

Katalysator A

Ein Aluminiumsilikatzeolith vom Pentasil-Typ wurde unter hydrothermalen Bedingungen bei autogenem Druck und 150 °C aus 650 g hochdispersem $SiO_2$, 203 g $Al_2(SO_4)_3$ x 18 $H_2O$ in 10 kg einer wäßrigen 1,6-Hexandiamin-Lösung (Mischung 50 : 50 Gew.-%) in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Aluminiumsilikatzeolith enthielt 92,8 Gew.-% $SiO_2$ und 4,2 Gew.-% $Al_2O_3$. Mit dieser Material werden durch Verformen mit einem Verformungshilfsmittel 2-mm-Stränge hergestellt, die bei 110 °C/16 h getrocknet und bei 500 °C /24 h calciniert werden.

Katalysator B

Ein Eisensilikatzeolith des Pentasil-Typs wird unter hydrothermalen Bedingungen bei autogenem Druck und 165 °C aus 273 g Wasserglas, gelöst ind 253 g einer wäßrigen 1,6-Hexandiamin-Lösung (Mischung 50

: 50 Gew.-%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolithen mit einem $SiO_2/Fe_2O_3$-Verhältnis von 17,7 und einem $Na_2O$-Gehalt von 1,2 Gew.-%. Der Zeolith wird mit hochdispersem $SiO_2$ im Gewichtsverhältnis 80 : 20 zu 2,5-mm-Strängen verstrangt, bei 110 °C/16 h getrocknet und bei 500 °C/24 h calciniert. Danach wird mit einer 20%igen $NH_4Cl$-Lösung bei 80 °C ionenausgetauscht, solange bis der Na-Gehalt nach der Trocknung bei 110 °C und Calcination bei 500 °C/5 h 0,002 Gew.-% beträgt.

Katalysator C

Katalysator C wird erhalten durch Verformung des Aluminiumsilikatzeolithen (vgl. Katalysator A) mit $SiO_2$ (Gewichtsverhältnis 80 : 20) zu 2-mm-Strängen, die bei 110 °C/16 h getrocknet und bei 500 °C/16 h calciniert werden.

Katalysator D

Katalysator D wird erhalten durch Verformung des Borsilikatzeolithen (vgl. Katalysator G) mit Boehmit (Gewichtsverhältnis 60 : 40) zu 2-mm-Strängen, die bei 110 °C/16 h getrocknet und bei 500 °C/16 h calciniert werden.

Katalysator E

Katalysator E wird erhalten durch Verformung des Borsilikatzeolithen (vgl. Katalysator G) mit $TiO_2$ - (Gewichtsverhältnis 60 : 40) zu 2-mm-Strängen, die bei 110 °C/ 16 h getrocknet und bei 500 °C /16 h calciniert werden.

Katalysator F

Zur Synthese des Katalysators F werden folgende Einsatzstoffe verwendet:
16.3 kg Wasser, 9.45 kg Natrium-Wasserglas, 0.27 kg Aluminiumsulfat-18-hydrat, 0.9 kg Schwefelsäure konz. und 0.9 kg 1,8-Diaminooctan. Das Aluminiumsulfat wird in Wasser gelöst, die Lösungen von Schwefelsäure und von Amin in Wasser vorsichtig zugegeben. Dann wird das Wasserglas zugefügt. Nach 120 h bei 160 °C wird der Kontakt wie bei Katalysator A beschrieben aufgearbeitet.

Katalysator G

Ein Borsilikatzeolith des Pentasil-Typs wird in einer hydrothermalen Synthese aus 640 g hochdispersem SiO2, 122 g HB03, 8000 g einer wäßrigen 1,6-Hexandiamin-Lösung (Mischung 50 : 50 Gew.-%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borsilikatzeolith setzt sich zusammen aus 94,2 Gew.-% $SiO_2$ und 2,3 Gew.-% $B_2O_3$. Daraus werden durch Verformen mit Boehmit im Gewichtsverhältnis 60 : 40 2 mm-Stränge hergestellt, die bei 110 °C/ 6 h getrocknet und bei 500 °C/24 h calciniert werden.

Katalysator H

Katalysator H wird aus Katalysator G hergestellt, durch Tränkung mit einer wäßrigen Lösung aus Ce-Nitrat. Der Ce-Gehalt beträgt 7.1 Gew.-%.

Katalysator I

Katalysator G ist kommerziell erhältliches Aluminiumoxid in Strangform.

Katalysator K

66 g Natriumhydroxid und 614.0 g Natriumgallat-Lösung (12,2 % Na, 12,7 % $Ga_2O_3$) werden in 100 g Wasser gelöst (Lösung A). Dann werden 1,5 kg hochdisperses $SiO_2$ in 24 kg Wasser suspendiert. 2,5 kg Tetrapropylammoniumhydroxid-Lösung 20 %-ig wird eingerührt. Anschließend wird die Lösung A hinzugefügt. Die Reaktionszeit beträgt 96 Stunden, die Reaktionstemperatur 175° C. Die Aufarbeitung erfolgt wie bei Katalysator A beschrieben. Dieser Galliumsilkatzeolith enthielt 96,4 Gew.% $SiO_2$ und 3,6 Gew.% $Ga_2O_3$.

Katalysator L

Katalysator L wird wie Katalysator K hergestellt, jedoch mit $SiO_2$ (Gewichtsverhältnis 80 : 20) verstrangt zu 2-mm-Strängen, die bei 110° C/16 h getrocknet und bei 500° C/16 h calciniert werden. Dieser Galliumsilikatzeolith enthielt 77,1 Gew.% $SiO_2$ und 2,9 Gew.% $Ga_2O_3$.

Beispiele

Beispiel 1 bis 24

Als Ausgangangssubstanzen werden 1,2-Diaminethan (Ethylendiamin ≙ EDA) und gegebenenfalls zusätzlich Piperazin eingesetzt, die WHSV beträgt ca. 3 $h^{-1}$. Die Reaktion wird unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

Aus den folgenden Tabellen I bis III sind die Reaktionstemperaturen, die Umsätze bezogen auf eingesetztes EDA und Selektivitäten zum 1,4-Diazabicyclo-2,2,2-octan enthalten.

Tabelle I

Herstellung von 1,4-Diazabicyclo(2,2,2)-octan (DAB) aus EDA. Ausgangssubstanz EDA 50 Gew.-% in Wasser. WHSV = 3 $h^{-1}$ falls nicht anders angegeben.

| Beispiel | Katalysator | Temperatur (° C) | Ausbeute (%) DAB | Ausbeute (%) Piperazin | |
|---|---|---|---|---|---|
| 1 | A | 350 | 47 | 10 | |
| 2 | A | 350 | 33 | 9 | WHSV = 24$h^{-1}$ |
| 3 | A | 400 | 43 | 11 | |
| 4 | C | 400 | 49 | 17 | |
| 5 | D | 400 | 32 | 22 | |
| 6 | E | 400 | 34 | 28 | |
| 7 | F | 400 | 41 | 13 | |
| 8 | F | 350 | 45 | 27 | |
| 9 | H | 400 | 43 | 36 | |
| 10 | I | 400 | 5 | 3 | |
| 11 | K | 400 | 43 | 16 | |
| 12 | L | 400 | 60 | 28 | |

Tabelle II

| Herstellung von 1,4-Diazabicyclo-2,2,2-octan (DAB) aus EDA. Ausgangssubstanz EDA rein. WHSV = 3 $h^{-1}$ | | | | |
|---|---|---|---|---|
| Beispiel | Katalysator | Temperatur (°C) | Ausbeute (%) | |
| | | | DAB | Piperazin |
| 13 | A | 400 | 35 | 11 |
| 14 | A | 350 | 19 | 10 |
| 15 | B | 350 | 28 | 15 |
| 16 | I | 350 | 1 | 3 |
| 17 | K | 400 | 49 | 11 |
| 18 | L | 400 | 48 | 12 |

Tabelle III

| Herstellung von 1,4-Diazabicyclo-2,2,2-octan aus EDA und Piperazin. Ausgangssubstanz EDA / Piperazin = 1/1 und 2/1 molar. WHSV = 3 $h^{-1}$. | | | | |
|---|---|---|---|---|
| Beispiel | Katalysator | Temperatur (°C) | Ausbeute 1,4-Diazabicyclo-2,2,2-octan (%) (Eduktgemisch Piperazin/ | |
| | | | EDA = 1/1 | 1/2 molar) |
| 19/20 | B | 400 | 55 | 70 |
| 21/22 | G | 400 | 41 | 60 |
| 23/24 | G | 350 | 14 | 27 |

## Ansprüche

1. Verfahren zur Herstellung von 1,4-Diazabicyclo-2,2,2-octan der Formel I

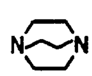

(I),

dadurch gekennzeichnet, daß man 1,2-Diaminoethan und 0 bis 200 Mol% Piperazin an Aluminium-, Bor-, Gallium- und/oder Eisensilikatzeolithen bei Temperaturen von 50 bis 600 °C und Drücken von 0,01 bis 50 bar umsetzt.

2. Verfahren nach Anspruch 1, dadurch gekennzeichnet, daß man die Umsetzung mit 30 bis 150 Mol% Piperazin durchführt.

3. Verfahren nach Anspruch 1, dadurch gekennzeichnet, daß man die Umsetzung mit 50 bis 100 Mol% Piperazin durchführt.

4. Verfahren nach Anspruch 1, dadurch gekennzeichnet, daß man die Umsetzung mit Aluminium-, Bor-, Gallium- und/oder Eisensilikatzeolithen des Pentasil-Typs durchführt.

5. Verfahren nach Anspruch 1, dadurch gekennzeichnet, daß man die Umsetzung an mit Seltenen Erden modifizierten Aluminium-, Bor-, Gallium- und/oder Eisensilikatzeolithen durchführt.

6. Verfahren nach Anspruch 1, dadurch gekennzeichnet, daß man die Umsetzung an mit Übergangsmetallen modifizierten Aluminium-, Bor-, Gallium- und/oder Eisensilikatzeolithen durchführt.

7. Verfahren nach Anspruch 1, dadurch gekennzeichnet, daß man die Reaktionen in der Gasphase bei

100 bis 600 °C und 0,01 bis 50 bar durchführt.

Europäisches Patentamt

# EUROPÄISCHER RECHERCHENBERICHT

Nummer der Anmeldung

EP 90101791.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| P,X | DE - A1 - 3 735 212 (HÜLS AG) * Ansprüche 1,4,5; Seiten 2,3 * -- | 1,4-7 | C 07 D 487/08// (C 07 D 487/08 C 07 D 241:00 C 07 D 241:00) |
| X | DE - A1 - 2 434 913 (SHUNAN PETROCHEMICAL CO. LTD.) * Ansprüche 1-5 * -- | 1,7 | |
| X | EP - A2 - 0 158 319 (UNION CARBIDE CORPORATION) * Anspruch 1; Seiten 6,12 * -- | 1,7 | |
| A | EP - A2 - 0 290 862 (BASF AKTIENGESELLSCHAFT) * Ansprüche 1,3-5,7; Seite 3 * -- | 1-7 | |
| A | EP - A2 - 0 263 463 (BASF AKTIENGESELLSCHAFT) * Ansprüche 1,3,4; Seiten 3,6 * -- | 1-7 | RECHERCHIERTE SACHGEBIETE (Int Cl.⁵) |
| P,A | EP - A2 - 0 349 859 (BASF AKTIENGESELLSCHAFT) * Anspruch; Seiten 3,4 * -- | 1-7 | C 07 D 487/00 |
| P,A | EP - A1 - 0 312 734 (HÜLS AKTIENGESELLSCHAFT) * Ansprüche 1-3,6; Seiten 3,4 * ---- | 1-7 | |

Der vorliegende Recherchenbericht wurde für alle Patentansprüche erstellt.

| Recherchenort | Abschlußdatum der Recherche | Prüfer |
|---|---|---|
| WIEN | 12-04-1990 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82